# NOUVEAU FASCICULE DE BREVET EUROPEEN

(11) **EP 0 734 729 B2**
(45) Date de publication et mention de la décision concernant l'opposition: **29.09.2004**
(45) Mention de la délivrance du brevet: 08.07.1998
(21) Numéro de dépôt: 96400457.6
(22) Date de dépôt: 04.03.1996
(51) Int. Cl.: A61K 38/22, A61K 39/395, A61K 7/48

(54) **Utilisation d'un antagoniste de CGRP pour traiter les rougeurs cutanées d'origine neurogène et composition obtenue**
Verwendung von einem CGRP Antagonist für die Behandlung von Hauterythemen neurogenischen Ursprungs und so erhaltene Zubereitungen
Use of a CGRP antagonist for the treatment of cutaneous erythema of neurogenic origin and so obtained composition

(30) Priorité: 28.03.1995 FR 9503628
(43) Date de publication de la demande: 02.10.1996
(73) Titulaire: L'OREAL, 75008 Paris (FR)
(72) Inventeur: De Lacharriere, Olivier, F-75015 Paris (FR); Breton, Lionel, F-78000 Versailles (FR)
(74) Mandataire: Lhoste, Catherine

(56) Documents cités:
- WO-A-93/21911
- NEUROSCIENCE, vol. 48, no. 4, Juin 1992, pages 963-968, XP000577183 T.L. BUCKLEY ET AL.: "THE PARTIAL INHIBITION OF INFLAMMATORY RESPONSES INDUCED BY A CAPSAICIN USING THE FAB FRAGMENT OF A SELECTIVE CALCITONIN GENE-RELATED PEPTIDE ANTISERUM IN RABBIT SKIN"
- BRITISH JOURNAL OF PHARMACOLOGY, vol. 110, no. 2, 1993, pages 772-776, XP000563605 K. JANE ESCOTT ET AL.: "EFFECT OF CALCITONIN GENE-RELATED PEPTIDE ANTAGONIST (CGRP 8-37) ON SKIN VASODILATATION AND OEDEMA INDUCED BY STIMULATION OF THE RAT SAPHENOUS NERVE"
- BRITISH JOURNAL OF PHARMACOLOGY, vol. 104, no. 3, Novembre 1991, pages 738-742, XP000563606 S.R. HUGHES ET AL.: "A CALCITONIN GENE-RELATED PEPTIDE (CGRP) ANTAGONIST (CGRP 8-37) INHIBITS MICROVASCULAR REPONSES INDUCED BY CGRP AND CAPSAICIN IN SKIN."
- NEUROSCIENCE LETTERS, vol. 102, no. 2.3, 31 Juillet 1989, pages 257-260, XP000578094 S.M. LOUIS ET AL.: "ANTIBODIES TO CALCITONIN-GENE RELATED PEPTIDE REDUCE INFLAMMATION INDUCED BY TOPICAL MUSTARD OIL BUT NOT THAT DUE TO CARRAGEENIN IN THE RAT"
- Dermatology 1993, 187, 153-158
- Pschyrembel, Klinisches Wörterbuch, Walter de Gruyter 1994, 435
- Pathol. Biol. (Paris) 1993, 41(10) 936-942
- Eur. J. Pharmacol 1991, 192(1), 85-88
- Br. J. Pharmacol. Vol. 103, 1991, 1515-1519
- The Journal of Dermatology, 1981, 8, 323-327
- Dictionnaire des termes de médecine, Garnier Delamare, 22ème ed., 1989, 299
- Dermatologie und Venerologie, Springer Verlag, 1984,367, 551-553, 644-647
- The Journal of Investigative Dermatology, Vol. 87, No.4,1986, 533-536
- Clinics in Dermatology 1993, 11:225-234
- The Journal of Immunology, Vol. 146, No.10,1991, 3424-3430
- British Medical Journal, vol.287, 1983, 1427-1428
- British Journal of Dermatology, 1983, 108, 111-113

## Description

La présente invention se rapporte à l'utilisation d'un antagoniste de CGRP (peptide dérivé du gène de la calcitonine : Calcitonin Gene Related Peptide en langue anglosaxonne) pour la préparation d'une composition cosmétique, pharmaceutique ou dermatologique, à application topique, destinée à traiter les rougeurs cutanées d'origine neurogène, les dites rougeurs cutanées d'origine neurogène étant celles de la rosacée et/ou de l'érythème pudique.

Plus spécialement, cette composition permet le traitement par voie topique, des rougeurs cutanées d'origine neurogène de la rosacée ou de l'érythème pudique.

La rosacée est une affection cutanée caractérisée par un érythème du visage prédominant sur les pommettes, le front, le nez, une hyperséborrhée du visage au niveau du front, du nez et des joues, et par une composante infectieuse avec des pustules acnéiformes.

Par ailleurs, il s'associe à ces signes une composante neurogène, c'est-à-dire une hyperréactivité cutanée de la peau du visage et du cou, caractérisée par l'apparition de rougeurs et de sensations subjectives du type démangeaisons ou prurit, de sensations de brûlures ou d'échauffement, de sensations de picotements, de fourmillements, d'inconforts, de tiraillements, etc.

Ces signes d'hyperréactivité peuvent être déclenchés par des facteurs très divers tels que la prise d'aliments, de boissons chaudes ou alcoolisées, par des variations de température rapide, par la chaleur et notamment l'exposition aux ultraviolets ou aux infrarouges, par une humidité relative basse, par l'exposition de la peau aux vents violents ou aux courants d'air (soufflerie, air conditionné), par l'application de tensio-actifs, de topiques dermatologiques irritants (rétinoïdes, peroxyde de benzoyle, alpha-hydroxy-acides...) ou l'utilisation de certains cosmétiques même lorsque ceux-ci ne sont pas connus comme particulièrement irritants.

Jusqu'alors, le mécanisme de déclenchement de ces signes était très mal connu et la rosacée était traitée par des actifs tels que les antiséborrhéiques et les antiinfectieux, par exemple le peroxyde de benzoyle, l'acide rétinoïque, le métronidazol ou les cyclines, qui agissaient sur l'infection et l'hyperséborrhée, mais ne permettaient pas de traiter la composante neurogène de cette affection, et notamment l'hyperréactivité de la peau et la rougeur.

De même, il n'existait pas jusqu'à présent de traitement pour la rougeur apparaissant dans l'érythème pudique. Celui-ci survient lors d'une émotion et se caractérise par une rougeur du visage et du décolleté, qui peut éventuellement s'accompagner d'un prurit (démangeaisons). Cette affection est très gênante pour les personnes qui en souffrent, et jusqu'alors on ne pouvait la traiter que par des bêta-bloquants, médicaments puissants utilisés pour traiter l'hyper-tension et présentant de nombreuses contre-indications.

Il subsiste donc le besoin d'un traitement de la rougeur cutanée et de l'état d'hyperréactivité de la peau atteinte de rosacée ou d'érythème pudique.

La présente invention a justement pour objet l'utilisation d'un ou plusieurs antagonistes de CGRP pour traiter les rougeurs cutanées d'origine neurogène de ces affections de la rosacée et/ou de l'erythème pudique.

Le CGRP est un élément chimique polypeptidique élaboré et libéré par une terminaison nerveuse. Le CGRP intervient notamment dans des maladies respiratoires et inflammatoires, dans des maladies allergiques et dans certaines maladies dermatologiques telles que l'eczéma ou le prurigo.

Cependant, personne n'avait envisagé jusqu'à ce jour de les utiliser pour traiter la rougeur cutanée d'origine neurogène.

La demanderesse a maintenant découvert que l'utilisation d'antagonistes de CGRP pouvait permettre d'obtenir un effet préventif et/ou curatif des rougeurs cutanées.

La présente invention a donc pour objet l'utilisation d'au moins un antagoniste de CGRP pour la préparation d'une composition cosmétique, pharmaceutique ou dermatologique, destinée à traiter les rougeurs cutanées d'origine neurogène, les dites rougeurs cutanées d'origine neurogène étant celles de la rosacée et/ou de l'erythème pudique notamment due à la libération de neuropeptides.

L'application de compositions contenant un ou des antagonistes de CGRP permet d'obtenir une nette diminution voire une disparition complète de la rougeur qui se manifeste aussi bien dans la rosacée que dans l'érythème pudique.

L'antagoniste de CGRP agit donc sur la composante neurogène de ces affections, pour laquelle on n'avait pas de traitement jusqu'à présent, et renforce ainsi l'efficacité des actifs utilisés jusqu'à présent pour le traitement de leur composante infectieuse, notamment dans le cas de la rosacée.

La composition de l'invention peut être appliquée par voie locale, c'est-à-dire par voie topique

La présente invention a aussi pour objet un procédé de traitement cosmétique, des rougeurs cutanées d'origine neurogène, les dites rougeurs cutanées d'origine neurogène étant celles de l'érythème pudique, caractérisé par le fait que l'on applique sur la peau, sur le cuir chevelu, et/ou sur les muqueuses, une composition contenant au moins un antagoniste de CGRP dans un milieu cosmétiquement, acceptable.

La composition de l'invention destinée à l'application topique contient un milieu cosmétiquement, pharmaceutiquement ou dermatologiquement acceptable, c'est-à-dire un milieu compatible avec la peau, les ongles, les muqueuses, les tissus et les cheveux. La composition contenant l'antagoniste de CGRP peut être appliquée par voie topique sur le visage, le cou, les cheveux, les muqueuses et les ongles, les grands plis ou toute autre zone cutanée du corps.

La demanderesse définit un antagoniste de CGRP comme toute molécule d'origine organique ou minérale capable de produire une inhibition de la fixation réceptorielle du CGRP ou de produire une inhibition de la synthèse et/ou de la libération de CGRP par les fibres nerveuses sensitives.

Pour qu'une substance soit reconnue comme un antagoniste de CGRP, elle doit répondre notamment à la caractéristique suivante : avoir une activité pharmacologique antagoniste du CGRP, c'est-à-dire induire une réponse pharmacologique cohérente notamment dans l'un des tests suivants :
- la substance antagoniste doit diminuer la vasodilatation induite par la capsaïcine et/ou
- la substance antagoniste doit provoquer une inhibition de la libération de CGRP par les fibres nerveuses sensitives et/ou

En outre, l'antagoniste peut avoir une affinité pour les récepteurs au CGRP.

On peut utiliser dans l'invention par exemple comme antagoniste de CGRP, le CGRP 8-37, un anticorps anti-CGRP.

Dans les compositions selon l'invention, l'antagoniste de CGRP est utilisé de préférence en une quantité allant de 0,000001 à 10 % en poids par rapport au poids total de la composition, et en particulier en une quantité allant de 0,0001 à 5 % en poids par rapport au poids total de la composition.

De façon avantageuse, on peut associer à l'antagoniste de CGRP un ou plusieurs antagonistes d'un autre neuropeptide comme des antagonistes de substance P et/ou un ou plusieurs antagonistes de médiateur de l'inflammation comme des antagonistes d'histamine, des antagonistes d'interleukine 1 (1 L1), et des antagonistes de Tumor Necrosis Factor alpha (TNF α).

Aussi, l'invention a encore pour objet une composition cosmétique, pharmaceutique et/ou dermatologique pour traiter les rougeurs cutanées, caractérisée en ce qu'elle contient, dans un milieu cosmétiquement, pharmaceutiquement et/ou dermatologiquement acceptable, au moins un antagoniste de CGRP et au moins un antagoniste d'un autre neuropeptide et/ou au moins un antagoniste de médiateur de l'inflammation.

L'antagoniste de neuropeptide autre que le CGRP est de préférence un antagoniste de substance P.

La substance P est un élément chimique polypeptidique élaboré et libéré par une terminaison nerveuse. Elle fait partie de la famille des tachykinines qui proviennent des terminaisons nerveuses libres de l'épiderme et du derme. La substance P intervient notamment dans la transmission de la douleur et dans des maladies du système nerveux central tels que l'anxiété, la schizophrénie, dans des maladies respiratoires et inflammatoires, dans des maladies gastrointestinales, dans des maladies rhumatismales et dans certaines maladies dermatologiques telles que l'eczéma, le psoriasis, l'urticaire et les dermites de contact.

Pour qu'une substance soit reconnue comme un antagoniste de substance P, elle doit répondre à la caractéristique suivante :
- avoir une activité pharmacologique antagoniste de la substance P, c'est-à-dire induire une réponse pharmacologique cohérente dans au moins l'un des deux tests suivants :
   - la substance antagoniste doit diminuer l'extravasation du plasma au travers de la paroi vasculaire induite par la capsaïcine ou par une stimulation nerveuse antidromique, ou bien
   - la substance antagoniste doit provoquer une inhibition de la contraction des muscles lisses induites par l'administration de substance P.

L'antagoniste de substance P peut en outre avoir une affinité sélective pour les récepteurs NK1 des tachykinines.

L'antagoniste de substance P de l'invention peut être fonctionnel ou réceptoriel, c'est-à-dire inhiber la synthèse et/ou la libération de substance P, ou empêcher sa fixation et/ou moduler son action.

L'antagoniste de substance P est de préférence un antagoniste réceptoriel de substance P.

L'antagoniste de substance P de l'invention peut être choisi parmi les peptides ou les dérivés non peptidiques, et plus précisément ceux comportant un hétérocycle azoté, soufré ou oxygéné, ou les composés azotés comportant un atome d'azote lié directement ou indirectement à un cycle benzénique.

On peut utiliser dans l'invention par exemple comme peptide antagoniste de substance P, le sendide et le spantide II.

On peut également utiliser dans invention comme peptide ceux décrits dans les documents US-A-4472305, US-A-4839465, EP-A-101929, EP-A-333174, EP-A-336230, EP-A-394989, EP-A-443132, EP-A-498069, EP-A-515681, EP-A-517589, WO-A-92/22569 et GB-A-2216529.

Les antagonistes de substance P non peptidiques utilisables dans l'invention sont notamment des composés hétérocycliques notamment soufrés, azotés ou oxygénés ou des composés comprenant un atome d'azote lié directement ou indirectement à un cycle benzénique.

Comme composé hétérocyclique, on peut utiliser dans l'invention ceux décrits dans les documents suivants : EP-A-360390, EP-A-429366, EP-A-430771, EP-A-499313, EP-A-514273, EP-A-514274, EP-A-514275, EP-A-514276, EP-A-520555, EP-A- 528495, EP-A-532456, EP-A-545478, EP-A-558156, WO-A-90/05525, WO-A-90/05729, WO-A-91/18878, WO-A-91/18899, WO-A-92/12151, WO-A-92/15585, WO-A-92/17449, WO-A-92/20676, WO-A-93/00330, WO-A-93/00331, WO-A-93/01159, WO-A-93/01169, WO-A-93/01170, WO-A-93/06099, WO-A-93/09116, WO-A-94/08997. En particulier, le composé comprenant au moins un hétérocycle azoté est un dérivé de 2-tricyclyl-2-amino-éthane, un dérivé de spirolactame, un dérivé de quinuclidine, un dérivé azacyclique, un dérivé d'aminopyrrolidine, un dérivé de pipéridine, un aminoazahétérocycle, un dérivé d'isoindole.

Comme composés comportant un atome d'azote lié directement ou indirectement à un noyau benzénique, on peut citer ceux décrits dans les documents suivants : EP-A-522808, WO-A-93/01165.

Comme antagonistes de médiateur de 'inflammation utilisables dans l'invention, on peut citer les dérivés de la diéthylène diamine telle que cinnarizine, cyclizine ; les dérivés de l'aminopropane (dexchlorophéniramine, tripolidine) ; des dérivés de phénothiazine (alimémazine, prométhazine) ; l'auranofine ; la lisophyline ; l'A802715 ; la sulfasalazine ; la cétirizine HCI ; la loratidine ; l'esbatine ; la sétastine HCl.

A titre d'exemple, les antagonistes de substance P et les antagonistes de médiateur de l'inflammation peuvent être utilisés en une quantité représentant de 0,000001 à 10 % du poids total de la composition et mieux de 0,0001 à 5 %.

Les compositions selon l'invention peuvent se présenter sous toutes les formes galéniques normalement utilisées selon la voie d'utilisation topique.

Pour une application topique, la composition peut se présenter notamment sous toutes les formes galéniques normalement utilisées pour une telle application, notamment sous forme de solutions aqueuses, hydroalcooliques ou huileuses, de dispersions du type lotion ou sérum, de gels aqueux, anhydres ou lipophiles, d'émulsions de consistance liquide ou semi-liquide du type lait, obtenues par dispersion d'une phase grasse dans une phase aqueuse (H/E) ou inversement (E/H), ou de suspensions ou émulsions de consistance molle, semi-solide ou solide du type crème ou gel, ou encore de microémulsions, de microcapsules, de microparticules ou de dispersions vésiculaires de type ionique et/ou non ionique. Ces compositions sont préparées selon les méthodes usuelles.

Elles peuvent être également utilisées pour les cheveux sous forme de solutions aqueuses, alcooliques ou hydroalcooliques, ou sous forme de crèmes, de gels, d'émulsions, de mousses ou encore sous forme de compositions pour aérosol contenant également un agent propulseur sous pression.

Les quantités des différents constituants des compositions selon l'invention sont celles classiquement utilisées dans les domaines considérés.

Ces compositions constituent notamment des crèmes de protection, de traitement ou de soin pour le visage, pour les mains, pour les pieds, pour les grands plis anatomiques ou pour le corps, des laits corporels de protection ou de soin, des lotions, gels ou mousses pour le soin de la peau et des muqueuses, comme des lotions de nettoyage ou de désinfection, des compositions pour le bain, des compositions contenant un agent bactéricide.

Les compositions peuvent également consister en des préparations solides constituant des savons ou des pains de nettoyage.

Les quantités des différents constituants des compositions selon l'invention sont celles classiquement utilisées dans les domaines considérés.

Le procédé de traitement cosmétique de l'invention peut être mis en oeuvre notamment en appliquant les compositions hygiéniques ou cosmétiques telles que définies ci-dessus, selon la technique d'utilisation habituelle de ces compositions. Par exemple : application de crèmes, de gels, de sérums, de lotions, de laits sur la peau, le cuir chevelu et/ou les muqueuses.

Lorsque la composition de l'invention est une émulsion, la proportion de la phase grasse peut aller de 5 % à 80 % en poids, et de préférence de 5 % à 50 % en poids par rapport au poids total de la composition. Les huiles, les émulsionnants et les coémulsionnants utilisés dans la composition sous forme d'émulsion sont choisis parmi ceux classiquement utilisés dans le domaine cosmétique. L'émulsionnant et le coémulsionnant sont présents, dans la composition, en une proportion allant de 0,3 % à 30 % en poids, et de préférence de 0,5 à 20 % en poids par rapport au poids total de la composition. L'émulsion peut, en outre, contenir des vésicules lipidiques.

Lorsque la composition de l'invention est une solution ou un gel huileux, la phase grasse peut représenter plus de 90 % du poids total de la composition.

De façon connue, la composition cosmétique, pharmaceutique ou dermatologique de l'invention peut contenir également des adjuvants habituels dans les domaines considérés, tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur et les matières colorantes. Les quantités de ces différents adjuvants sont celles classiquement utilisées dans les domaines considérés, et par exemple de 0,01 % à 20 % du poids total de la composition. Ces adjuvants, selon leur nature, peuvent être introduits dans la phase grasse, dans la phase aqueuse et/ou dans les sphérules lipidiques.

Comme huiles utilisables dans l'invention, on peut citer les huiles minérales (huile de vaseline), les huiles végétales (fraction liquide du beurre de karité, huile de tournesol), les huiles animales (perhydrosqualène), les huiles de synthèse (huile de Purcellin), les huiles siliconées (cyclométhicone) et les huiles fluorées (perfluoropolyéthers). On peut aussi utiliser comme matières grasses des alcools gras, des acides gras (acide stéarique), des cires (paraffine, carnauba, cire d'abeilles).

Comme émulsionnants utilisables dans l'invention, on peut citer par exemple le stéarate de glycérol, le polysorbate 60 et le mélange de PEG-6/PEG-32/Glycol Stéarate vendu sous la dénomination de Tefose^{R} 63 par la société Gattefosse.

Comme solvants utilisables dans l'invention, on peut citer les alcools inférieurs, notamment l'éthanol et l'isopropanol, le propylène glycol.

Comme gélifiants hydrophiles, on peut citer les polymères carboxyvinyliques (carbomer), les copolymères acryliques tels que les copolymères d'acrylates/alkylacrylates, les polyacrylamides, les polysaccharides tels que l'hydroxypropylcellulose, les gommes naturelles et les argiles, et, comme gélifiants lipophiles, on peut citer les argiles modifiées comme les bentones, les sels métalliques d'acides gras comme les stéarates d'aluminium et la silice hydrophobe, ou encore l'éthylcellulose, le polyéthylène

Comme actifs hydrophiles, on peut utiliser les protéines ou les hydrolysats de protéine, les acides aminés, les polyols, l'urée, l'allantoïne, les sucres et les dérivés de sucre, les vitamines hydrosolubles, l'amidon et des extraits végétaux, notamment ceux d'Aloe Vera.

Comme actifs lipophiles, on peut utiliser le rétinol (vitamine A) et ses dérivés, le tocophérol (vitamine E) et ses dérivés, les acides gras essentiels, les céramides, les huiles essentielles.

On peut entre autres associer les antagonistes de CGRP à des agents actifs destinés notamment à la prévention et/ou au traitement des affections cutanées. Parmi ces agents actifs, on peut citer à titre d'exemple :
- les agents modulant la différenciation et/ou la prolifération et/ou la pigmentation cutanée tels que l'acide rétinoïque et ses isomères, le rétinol et ses esters, la vitamine D et ses dérivés, les estrogènes tels que l'estradiol, l'acide kojique ou l'hydroquinone ;
- les antibactériens tels que le phosphate de clindamycine, l'érythromycine ou les antibiotiques de la classe des tétracyclines ;
- les antiparasitaires, en particulier le métronidazole, le crotamiton ou les pyréthrinoïdes ;
- les antifongiques, en particulier les composés appartenant à la classe des imidazoles tels que l'éconazole, le kétoconazole ou le miconazole ou leurs sels, les composés polyènes, tels que l'amphotéricine B, les composés de la famille des allylamines, tels que la terbinafine, ou encore l'octopirox ;
- les agents anti-inflammatoires stéroïdiens, tels que l'hydrocortisone, le valérate de bétaméthasone ou le propionate de clobétasol, ou les agents anti-inflammatoires non-stéroïdiens tels que l'ibuprofène et ses sels, le diclofénac et ses sels, l'acide acétylsalicylique, l'acétaminophène ou l'acide glycyrrhétinique ;
- les agents anesthésiques tels que le chlorhydrate de lidocaïne et ses dérivés ;
- les agents antiprurigineux comme la thénaldine, la triméprazine ou la cyproheptadine ;
- les agents antiviraux tels que l'acyclovir ;
- les agents kératolytiques tels que les acides alpha- et bêta-hydroxycarboxyliques ou bêta-cétocarboxyliques, leurs sels, amides ou esters et plus particulièrement les hydroxyacides tels que l'acide glycolique, l'acide lactique, l'acide salicylique, l'acide citrique et de manière générale les acides de fruits, et l'acide n-octanoyl-5-salicylique ;
- les agents anti-radicaux libres, tels que l'alpha-tocophérol ou ses esters, les superoxyde dismutases, certains chélatants de métaux ou l'acide ascorbique et ses esters ;
- les antiséborrhéiques tels que la progestérone ;
- les antipelliculaires comme l'octopirox ou la pyrithione de zinc ;
- les antiacnéiques comme l'acide rétinoique ou le peroxyde de benzoyle.

Les exemples suivants illustrent l'invention. Dans ces exemples, les proportions indiquées sont des pourcentages en poids.

### Exemple 1 : Crème pour le visage (émulsion huile dans eau)

| | |
|---|---|
| CGRP 8-37 | 0,5 % |
| Stéarate de glycérol | 2 % |
| Polysorbate 60 (Tween 60 vendu par la société ICI) | 1 % |
| Acide stéarique | 1,4 % |
| Triéthanolamine | 0,7 % |
| Carbomer | 0,4 % |
| Cyclométhicone | 8 % |
| Huile de tournesol | 12 % |
| Antioxydant | 0,05 % |
| Conservateur | 0,3 % |
| Eau | qsp 100 % |

### Exemple 2 : Gel émulsionné (émulsion huile dans eau)

| | |
|---|---|
| Cyclométhicone | 3 % |
| Huile de Purcellin (vendue par la Société Dragocco) PEG-6/PEG-32/Glycol Stéarate (Tefose^{R} 63 de | 7 % |
| Gattefosse) | 0,3 % |
| CGRP 8-37 | 0,0001 |
| Conservateur | 0,3 % |
| Carbomer | 0,6 % |
| Crotamiton | 5 % |
| Acide glycyrrhétinique | 2 % |
| Alcool éthylique | 5 % |
| Triéthanolamine | 0,2 % |
| Eau | qsp 100 % |

### Exemple 3 : Gel

| | |
|---|---|
| Acide all trans rétinoïque | 0,05 % |
| Anticorps Anti-CGRP | 0,05 % |
| Hydroxypropylcellulose (Klucel H vendu par la société Hercules) | 1 % |
| Antioxydant | 0,05 % |
| Isopropanol | 40 % |
| Conservateur | 0,3 % |
| Eau | qsp 100% |

### Exemple 4 : Gel

L'exemple 4 est identique à l'exemple 3, à l'exception du fait qu'il contient en plus 0,3 % de sendide.

### Exemple 5 : Crème (émulsion huile dans eau)

| | |
|---|---|
| Stéarate de glycérol | 2 % |
| CGRP 8-37 | 0,02 % |
| Polysorbate 60 (Tween 60 vendu par la société ICI) | 1 % |
| Acide stéarique | 1,4 % |
| Métronidazole | 0,5 % |
| Triéthanolamine | 0,7 % |
| Carbomer | 0,4 % |
| Cyclométhicone | 8 % |
| Huile de tournesol | 10 % |
| Antioxydant | 0,05 % |
| Conservateur | 0,3 % |
| Eau | qsp 100 % |

### Exemple 6 : Crème pour le visage (émulsion huile dans eau)

| | |
|---|---|
| Stéarate de glycérol | 2 % |
| Spantide | 0,5 % |
| Anticorps Anti-CGRP | 0,02 % |
| Polysorbate 60 (Tween 60 vendu par la société ICI) | 1 % |
| Acide stéarique | 1,4 % |
| Triéthanolamine | 0,7 % |
| Carbomer | 0,4 % |
| Cyclométhicone | 8 % |
| Huile de tournesol | 12 % |
| Antioxydant | 0,05 % |
| Conservateur | 0,3 % |
| Eau | qsp 100 % |

### Exemple 7 : Crème pour le visage (émulsion huile dans eau)

| | |
|---|---|
| CGRP 8-37 | 0,25 |
| Stéarate de glycérol | 2,00 |
| Polysorbate 60 (Tween 60 vendu par la société ICI) | 1,00 |
| Acide stéarique | 1,40 |
| Métronidazole | 1,00 |
| Triéthanolamine | 0,70 |
| Carbomer | 0,40 |
| Fraction liquide du beurre de karité | 12,00 |
| Huile de vaseline | 12,00 |
| Antioxydant | 0,05 |
| Parfum | 0,50 |
| Conservateur | 0,30 |
| Eau | qsp 100% |

## Revendications

1. Utilisation d'au moins un antagoniste de CGRP pour la préparation d'une composition cosmétique, pharmaceutique et/ou dermatologique destinée à traiter les rougeurs cutanées d'origine neurogène, les dites rougeurs cutanées d'origine neurogène étant celles de la rosacée et/ou de l'érythème pudique, la composition se présentant sous une forme appropriée pour une application topique.

2. Utilisation selon la revendication précédente, **caractérisée en ce que** l'antagoniste de CGRP est une molécule qui diminue la vasodilatation induite par la capsaïcine.

3. Utilisation selon l'une des revendications précédentes, **caractérisée en ce que** l'antagoniste de CGRP est une molécule choisie parmi le CGRP 8-37 et les anticorps anti-CGRP.

4. Utilisation selon l'une quelconque des revendications précédentes, **caractérisée en ce que** l'antagoniste de CGRP est utilisé en une quantité allant de 0,000001 à 10 % en poids par rapport au poids total de la composition.

5. Utilisation selon l'une quelconque des revendications précédentes, **caractérisée en ce que** l'antagoniste de CGRP est utilisé en une quantité allant de 0,0001 à 5 % en poids par rapport au poids total de la composition.

6. Utilisation selon l'une quelconque des revendications précédentes, **caractérisée en ce que** la composition contient, en outre, au moins un agent choisi parmi les antagonistes de neuropeptides autres que le CGRP, les antagonistes de médiateur de l'inflammation, les agents antibactériens, antiparasitaires, antifongiques, anti-inflammatoires, antiprurigineux, anesthésiques, antiviraux, kératolytiques, anti-radicaux libres, antiséborrhéiques, antipelliculaires, antiacnéiques et/ou les agents modulant la différenciation et/ou la prolifération et/ou la pigmentation cutanée.

7. Utilisation selon l'une quelconque des revendications précédentes, **caractérisée en ce que** la composition contient, en outre, au moins un antagoniste de substance P, un antagoniste d'histamine, un antagoniste d'interleukine 1 et/ou un antagoniste de TNFα.

8. Utilisation selon la revendication 7, **caractérisée en ce que** l'antagoniste est utilisé en une quantité représentant de 0,0001 à 5 % du poids total de la composition.

9. Utilisation selon l'une quelconque des revendications précédentes, **caractérisée en ce que** la composition contient en outre au moins un actif choisi parmi les protéines ou les hydrolysats de protéine, les acides aminés, les polyols, l'urée, les sucres et les dérivés de sucre, les vitamines, l'amidon, les extraits végétaux, les acides gras essentiels, les céramides, les huiles essentielles, les hydroxyacides.

10. Utilisation selon l'une quelconque des revendications précédentes, **caractérisée en ce que** la composition est une solution aqueuse, huileuse ou hydroalcoolique, une émulsion eau-dans-huile, une émulsion huile-dans-eau, une microémulsion, un gel aqueux, un gel anhydre, un sérum, une dispersion de vésicules, de microcapsules ou de microparticules.

11. Composition cosmétique, pharmaceutique et/ou dermatologique pour traiter les rougeurs cutanées, **caractérisée en ce qu'**elle contient, dans un milieu cosmétiquement, pharmaceutiquement et/ou dermatologiquement acceptable, au moins un antagoniste de CGRP et au moins un antagoniste d'un autre neuropeptide et/ou au moins un antagoniste d'un médiateur de l'inflammation.

12. Composition selon la revendication 11, **caractérisée en ce que** l'antagoniste de CGRP est une molécule qui diminue la vasodilatation induite par la capsaïcine.

13. Composition selon l'une quelconques des revendications 11 et 12, **caractérisée en ce que** l'antagoniste de CGRP est une molécule choisie parmi le CGRP 8-37 et les anticorps anti-CGRP.

14. Composition selon l'une quelconques des revendications 11 à 13, **caractérisée en ce que** l'antagoniste de CGRP est utilisé en une quantité allant de 0,000001 à 10 % en poids par rapport au poids total de la composition.

15. Composition selon l'une quelconque des revendications 11 à 14, **caractérisée en ce que** l'antagoniste de CGRP est utilisé en une quantité allant de 0,0001 à 5 % en poids par rapport au poids total de la composition.

16. Composition selon l'une quelconque des revendications 11 à 15, **caractérisée en ce que** l'antagoniste du neuropeptide autre que le CGRP est choisi parmi les antagonistes de substance P.

17. Composition selon l'une quelconque des revendications 11 à 16, **caractérisée en ce que** l'antagoniste de substance P est un antagoniste réceptoriel de substance P.

18. Composition selon l'une quelconque des revendications 11 à 17, **caractérisée en ce que** l'antagoniste de médiateur de l'inflammation est choisi parmi les antagonistes d'histamine, les antagonistes d'interleukine 1 et les antagonistes de TNFα.

19. Composition selon l'une quelconque des revendications 11 à 18, **caractérisée en ce que** l'antagoniste de substance P ou l'antagoniste de médiateur de l'inflammation est utilisé en une quantité représentant de 0,000001 à 5 % du poids total de la composition.

20. Composition selon l'une quelconque des revendications 11 à 19, **caractérisée en ce qu'**elle contient en outre au moins un agent choisi parmi les agents antibactériens, antiparasitaires, antifongiques, anti-inflammatoires, antiseptiques, antiprurigineux, anesthésiques, antiviraux, kératolytiques, anti-radicaux libres, antiséborrhéiques, antipelliculaires, antiacnéiques et les agents modulant la différenciation et/ou la prolifération et/ou la pigmentation cutanée.

21. Composition selon l'une quelconque des revendications 11 à 20, **caractérisée en ce qu'**elle contient, en outre, des adjuvants tels que les gélifiants hydrophiles ou lipophiles, les actifs hydrophiles ou lipophiles, les conservateurs, les antioxydants, les solvants, les parfums, les charges, les filtres, les bactéricides, les absorbeurs d'odeur et les matières colorantes.

22. Composition selon l'une quelconque des revendications 11 à 21, **caractérisée en ce que** le milieu cosmétiquement, pharmaceutiquement et/ou dermatologiquement acceptable est une solution aqueuse, huileuse ou hydroalcoolique, une émulsion eau-dans-huile, une émulsion huile-dans-eau, une microémulsion, un gel aqueux, un gel anhydre, un sérum, une dispersion de vésicules.

23. Procédé de traitement cosmétique des rougeurs cutanées d'origine neurogène, les dites rougeurs cutanées d'origine neurogène étant celles de l'érythème pudique, **caractérisé par le fait que** l'on applique sur la peau, sur le cuir chevelu, et/ou sur les muqueuses, une composition contenant au moins un antagoniste de CGRP dans un milieu cosmétiquement acceptable.

## Patentansprüche

1. Verwendung mindestens eines Antagonisten von CGRP für die Herstellung einer kosmetischen, pharmazeutischen und/oder dermatologischen Zusammensetzung, die für die Behandlung der Hautrötungen neurogenen Ursprungs vorgesehen ist, wobei es sich bei den Hautrötungen neurogenen Ursprungs um die Rosacea und/ oder das Erröten handelt, wobei die Zusammensetzung in einer für die topische Anwendung geeigneten Form vorliegt.

2. Verwendung nach dem vorhergehende Anspruch, **dadurch gekennzeichnet, dass** der Antagonist von CGRP ein Molekül ist, das die durch Capsaicin ausgelöste Vasodilatation verringert.

3. Verwendung nach einem der vorhergehenden Ansprüche, **dadurch gekennzeichnet, dass** der Antagonist von CGRP ein Molekül ist, das unter CGRP 8-37 und Anti-CGRP-Antikörpern ausgewählt ist.

4. Verwendung nach einem der vorhergehenden Ansprüche, **dadurch gekennzeichnet, dass** der Antagonist von CGRP in einer Menge verwendet wird, die im Bereich von 0,000001 bis 10 Gew.-%, bezogen auf das Gesamtgewicht der Zusammensetzung, liegt.

5. Verwendung nach einem der vorhergehenden Ansprüche, **dadurch gekennzeichnet, dass** der Antagonist von CGRP in einer Menge verwendet wird, die im Bereich von 0,0001 bis 5 Gew.-%, bezogen auf das Gesamtgewicht der Zusammensetzung, liegt.

6. Verwendung nach einem der vorhergehenden Ansprüche, **dadurch gekennzeichnet, dass** die Zusammensetzung außerdem mindestens ein Mittel enthält, das unter den Antagonisten von Neuropeptiden, die von CGRP verschieden sind, den Antagonisten von Entzündungsmediatoren, antibakteriellen Mitteln, antiparasitären Mitteln, Fungiziden, entzündungshemmenden Mitteln, Antipruriginosa, Anästhetika, antiviralen Mitteln, Keratolytika, Mitteln gegen freie Radikale, Antiseborrhöika, Antischuppenmitteln, Aknemitteln und/oder Mitteln, die die Differenzierung und/oder die Proliferation und/oder die Pigmentierung der Haut modulieren, ausgewählt wird.

7. Verwendung nach einem der vorhergehenden Ansprüche, **dadurch gekennzeichnet, dass** die Zusammensetzung außerdem mindestens einen Antagonisten der Substanz P, Antagonisten von Histamin, Antagonisten von Interleukin 1 und/oder Antagonisten von TNF-α enthält.

8. Verwendung nach Anspruch 7, **dadurch gekennzeichnet, dass** der Antagonist in einer Menge von 0,0001 bis 5 Gew.-% des Gesamtgewichts der Zusammensetzung verwendet wird.

9. Verwendung nach einem der vorhergehenden Ansprüche, **dadurch gekennzeichnet, dass** die Zusammensetzung außerdem mindestens einen Wirkstoff enthält, der unter Proteinen und Proteinhydrolysaten, Aminosäuren, Polyolen, Harnstoff, Zuckern und Zuckerderivaten, Vitaminen, Stärke, Pflanzenextrakten, essentiellen Fettsäuren, Ceramiden, etherischen Ölen, Hydroxysäuren ausgewählt wird.

10. Verwendung nach einem der vorhergehenden Ansprüche, **dadurch gekennzeichnet, dass** die Zusammensetzung eine wäßrige, ölige oder wäßrig-alkoholische Lösung, eine Wasser-in-Öl-Emulsion, eine Öl-in-Wasser-Emulsion, eine Mikroemulsion, ein wäßriges Gel, ein wasserfreies Gel, ein Serum, eine Dispersion von Vesikeln, Mikrokapseln oder Mikropartikeln ist.

11. Kosmetische, pharmazeutische und/oder dermatologische Zusammensetzung zur Behandlung von Hautrötungen, **dadurch gekennzeichnet, dass** sie in einem kosmetisch, pharmazeutisch und/oder dermatologisch akzeptablen Medium mindestens einen Antagonisten von CGRP und mindestens einen Antagonisten eines anderen Neuropeptids und/oder mindestens einen Antagonisten eines Entzündungsmediators enthält.

12. Zusammensetzung nach Anspruch 11, **dadurch gekennzeichnet, dass** der Antagonist von CGRP ein Molekül ist, das die durch Capsaicin ausgelöste Vasodilatation verringert.

13. Zusammensetzung nach einem der Ansprüche 11 und 12, **dadurch gekennzeichnet, dass** der Antagonist von CGRP ein Molekül ist, das unter CGRP 8-37 und den Anti-CGRP-Antikörpern ausgewählt ist.

14. Zusammensetzung nach einem der Ansprüche 11 bis 13, **dadurch gekennzeichnet, dass** der Antagonist von CGRP in einer Menge verwendet wird, die im Bereich von 0,000001 bis 10 Gew.-%, bezogen auf das Gesamtgewicht der Zusammensetzung, liegt.

15. Zusammensetzung nach einem der Ansprüche 11 bis 14, **dadurch gekennzeichnet, dass** der Antagonist von CGRP in einer Menge verwendet wird, die im Bereich von 0,0001 bis 5 Gew.-%, bezogen auf das Gesamtgewicht der Zusammensetzung, liegt.

16. Zusammensetzung nach einem der Ansprüche 11 bis 15, **dadurch gekennzeichnet, dass** der Antagonist des von CGRP verschiedenen Neuropeptids unter den Antagonisten der Substanz P ausgewählt ist.

17. Zusammensetzung nach einem der Ansprüche 11 bis 16, **dadurch gekennzeichnet, dass** der Antagonist der Substanz P ein rezeptorbezogener Antagonist der Substanz P ist.

18. Zusammensetzung nach einem der Ansprüche 11 bis 16, **dadurch gekennzeichnet, dass** der Antagonist des Entzündungsmediators unter den Antagonisten von Histamin, den Antagonisten von Interleukin 1 und den Antagonisten von TNF-α ausgewählt ist.

19. Zusammensetzung nach einem der Ansprüche 11 bis 18, **dadurch gekennzeichnet, dass** der Antagonist der Substanz P oder der Antagonist des Entzündungsmediators in einer Menge von 0,000001 bis 5 % des Gesamtgewichts der Zusammensetzung verwendet wird.

20. Zusammensetzung nach einem der Ansprüche 11 bis 19, **dadurch gekennzeichnet, dass** sie außerdem mindestens ein Mittel enthält, das unter antibakteriellen Mitteln, antiparasitären Mitteln, Fungiziden, entzündungshemmenden Mitteln, Antiseptika, Antipruriginosa, Anästhetika, antiviralen Mitteln, Keratolytika, Mitteln gegen freie Radikale, Antiseborrhöika, Antischuppenmitteln, Aknemitteln und/ oder Mitteln, die die Differenzierung und/oder die Proliferation und/oder die Pigmentierung der Haut modulieren, ausgewählt ist.

21. Zusammensetzung nach einem der Ansprüche 11 bis 20, **dadurch gekennzeichnet, dass** sie außerdem Hilfsstoffe, wie z.B. hydrophile oder lipophile Gelbildner, hydrophile oder lipophile Wirkstoffe, Konservierungsmittel, Antioxidantien, Lösemittel, Parfüms, Füllstoffe, Filter, Bakterizide, Geruchsabsorber und Farbstoffe, enthalten.

22. Zusammensetzung nach einem der Ansprüche 11 bis 21, **dadurch gekennzeichnet, dass** das kosmetisch, pharmazeutisch und/oder dermatologisch akzeptable Medium eine wäßrige, ölige oder wäßrig-alkoholische Lösung, eine Wasser-in-Öl-Emulsion, eine Öl-in-Wasser-Emulsion, eine Mikroemulsion, ein wäßriges Gel, ein wasserfreies Gel, ein Serum, eine Vesikeldispersion ist.

23. Verfahren zur kosmetischen Behandlung von Hautrötungen neurogenen Ursprungs, wobei es sich bei den Hautrötungen neurogenen Ursprungs um das Erröten handelt, **dadurch gekennzeichnet, dass** auf die Haut, die Kopfhaut und/oder die Schleimhäute eine Zusammensetzung aufgetragen wird, die mindestens einen Antagonisten von CGRP in einem kosmetisch akzeptablen Medium enthält.

## Claims

1. Use of at least one CGRP antagonist for the preparation of a cosmetic, pharmaceutical and/or dermatological composition intended for treating skin redness of neurogenic origin, the said skin redness of neurogenic origin being that of rosacea and/or of discreet erythema, the composition being in a form that is suitable for topical application.

2. Use according to Claim 1, **characterized in that** the CGRP antagonist is a molecule which reduces the vasodilation induced by capsaicin.

3. Use according to either of the preceding claims, **characterized in that** the CGRP antagonist is a molecule chosen from CGRP 8-37 and anti-CGRP antibodies.

4. Use according to any one of the preceding claims, **characterized in that** the CGRP antagonist is used in an amount ranging from 0.000001 to 10 % by weight relative to the total weight of the composition.

5. Use according to any one of the preceding claims, **characterized in that** the CGRP antagonist is used in an amount ranging from 0.0001 to 5 % by weight relative to the total weight of the composition.

6. Use according to any one of the preceding claims, **characterized in that** the composition also contains at least one agent chosen from antagonists of neuropeptides other than CGRP, inflammation mediator antagonists, antibacterial agents, antiparasitic agents, antifungal agents, anti-inflammatory agents, antipruriginous agents, anaesthetics, antiviral agents, keratolytic agents, anti-free-radical agents, antiseborrhoeic agents, antidandruff agents, antiacne agents and/or agents which modify skin differentiation and/or proliferation and/or pigmentation.

7. Use according to any one of the preceding claims, **characterized in that** the composition also contains at least one substance P antagonist, a histamine antagonist, an interleukin 1 antagonist and/or a TNFα antagonist.

8. Use according to Claim 7, **characterized in that** the antagonist is used in an amount representing from 0.0001 to 5 % of the total weight of the composition.

9. Use according to any one of the preceding claims, **characterized in that** the composition also contains at least one active agent chosen from proteins or protein hydrolysates, amino acids, polyols, urea, sugars and sugar derivatives, vitamins, starch, plant extracts, essential fatty acids, ceramides, essential oils and hydroxy acids.

10. Use according to any one of the preceding claims, **characterized in that** the composition is an aqueous, oily or aqueous-alcoholic solution, a water-in-oil emulsion, an oil-in-water emulsion, a microemulsion, an aqueous gel, an anhydrous gel, a serum or a dispersion of vesicles, microcapsules or microparticles.

11. Cosmetic, pharmaceutical and/or dermatological composition for treating skin redness, **characterized in that** it contains, in a cosmetically, pharmaceutically and/or dermatologically acceptable medium, at least one CGRP antagonist and at least one antagonist of another neuropeptide and/or at least one inflammation mediator antagonist.

12. Composition according to Claim 11, **characterized in that** the CGRP antagonist is a molecule which reduces the vasodilation induced by capsaicin.

13. Composition according to either of Claims 11 and 12, **characterized in that** the CGRP antagonist is a molecule chosen from CGRP 8-37 and anti-CGRP antibodies.

14. Composition according to any one of Claims 11 to 13, **characterized in that** the CGRP antagonist is used in an amount ranging from 0.000001 to 10 % by weight relative to the total weight of the composition.

15. Composition according to any one of Claims 11 to 14, **characterized in that** the CGRP antagonist is used in an amount ranging from 0.0001 to 5 % by weight relative to the total weight of the composition.

16. Composition according to any one of Claims 11 to 15, **characterized in that** the antagonist of the neuropeptide other than CGRP is chosen from substance P antagonists.

17. Composition according to any one of Claims 11 to 16, **characterized in that** the substance P antagonist is a substance P receptor antagonist.

18. Composition according to any one of Claims 11 to 17, **characterized in that** the inflammation mediator antagonist is chosen from histamine antagonists, interleukin 1 antagonists and TNFα antagonists.

19. Composition according to any one of Claims 11 to 18, **characterized in that** the substance P antagonist or the inflammation mediator antagonist is used in an amount representing from 0.000001 to 5 % of the total weight of the composition.

20. Composition according to any one of Claims 11 to 19, **characterized in that** it also contains at least one agent chosen from antibacterial agents, antiparasitic agents, antifungal agents, anti-inflammatory agents, antiseptics, antipruriginous agents, anaesthetics, antiviral agents, keratolytic agents, anti-free-radical agents, antiseborrhoeic agents, antidandruff agents, antiacne agents and agents which modify skin differentiation and/or proliferation and/or pigmentation.

21. Composition according to any one of Claims 11 to 20, **characterized in that** it also contains adjuvants such as hydrophilic or lipophilic gelling agents, hydrophilic or lipophilic active agents, preserving agents, antioxidants, solvents, fragrances, fillers, screening agents, bactericides, odour absorbers and dyestuffs.

22. Composition according to any one of Claims 11 to 21, **characterized in that** the cosmetically, pharmaceutically and/or dermatologically acceptable medium is an aqueous, oily or aqueous-alcoholic solution, a water-in-oil emulsion, an oil-in-water emulsion, a microemulsion, an aqueous gel, an anhydrous gel, a serum or a vesicle dispersion.

23. Process for the cosmetic treatment of skin redness of neurogenic origin, the said skin redness of neurogenic origin being that of discreet erythema, **characterized in that** a composition containing at least one CGRP antagonist in a cosmetically acceptable medium is applied to the skin, to the scalp and/or to the mucous membranes.
